(19) Europäisches Patentamt
European Patent Office
Office européen des brevets

(11) Publication number: **0 247 724 B1**

(12) **EUROPEAN PATENT SPECIFICATION**

(45) Date of publication of patent specification: **11.09.91**

(51) Int. Cl.5: **C07D 417/04**, C07C 333/04, C07D 233/61

(21) Application number: **87303560.4**

(22) Date of filing: **23.04.87**

The file contains technical information submitted after the application was filed and not included in this specification

(54) **Thiadiazinon derivatives.**

(30) Priority: **28.04.86 GB 8610369**

(43) Date of publication of application:
**02.12.87 Bulletin 87/49**

(45) Publication of the grant of the patent:
**11.09.91 Bulletin 91/37**

(84) Designated Contracting States:
**AT BE CH DE ES FR GB GR IT LI LU NL SE**

(56) References cited:
**EP-A- 052 442**
**EP-A- 124 314**
**EP-A- 145 236**
**DE-A- 1 292 645**

**J. Med. Chem. 28, 1405 (1985)**

**CHEMICAL ABSTRACTS; vol.87, no.19, November 7, 1977, Columbus, Ohio, USA EGE, GUENTER; ARNOLD, PHILIPP; JOOSS, GERHARD; NORONHA, RUDOLF "Five-membered heterocycles. IV. Ring contraction of 5-aryl-6H-1, 3, 4-thiadiazin-2(3H)-ones to 4-aryl-1,2,3-thiadiazoles" page 591, column 2, abstract no.152 150h**

**CHEMICAL ABSTRACTS, vol. 91, no.17, October 22, 1979, Columbus, Ohio, USA EGE, GUENTER; ARNOLD, PHILIPP; NORONHA, RUDOLF "Five-membered ring heterocycles.VI. 3-(Organylamino)-1,3-thiazolin-2-ones and -2-thiones" page 651, column 2, abstract no. 140 761c**

(73) Proprietor: **SMITH KLINE & FRENCH LABORATORIES LIMITED**
**Mundells**
**Welwyn Garden City Hertfordshire, AL7 1EY(GB)**

(72) Inventor: **Coates, William John**
**45 Lodgefield**
**Welwyn Garden City Hertfordshire AL7 1SD(GB)**

(74) Representative: **Waters, David Martin, Dr. et al**
**Smith Kline & French Laboratories Ltd. Patent Department Mundells**
**Welwyn Garden City Hertfordshire AL7 1EY(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to heterocyclic compounds and in particular to such compounds having a thiadiazinone ring as part of a tricyclic structure. This invention further relates to processes for their preparation, their use as therapeutic agents and to pharmaceutical compositions containing them. The compounds of this invention are phosphodiesterase type III inhibitors and are of use in combatting such conditions wherein such inhibition is thought to be beneficial. Thus the compounds of this invention are positive inotropic agents and vasodilators and are therefore of value in combatting cardiovascular disease, in particular congestive heart failure. In addition the compounds of this invention inhibit platelet aggregation and therefore have an antithrombotic effect. Furthermore the compounds of this invention are bronchodilators and are therefore of use in combatting chronic obstructive lung diseases such as asthma and bronchitis. The major utility of the compounds of this invention is in the treatment of congestive heart failure, for such treatment the compounds have a very desirable profile of activity.

EP-A-145236 discloses indenothiadiazinone derivatives as inotropic agents. EP-A-124314 discloses indenopyridazinone derivatives as cardiotonic and antihypertensive agents. EP-A-52442 discloses phenyldiazinone derivatives as cardiotonic agents. Sircar et. al., J. Med. Chem., 1985, 28, 1405-1413 discloses imidazolylphenylpyridazinone derivatives as positive inotropic agents.

Accordingly the present invention provides compounds of the formula (1) :-

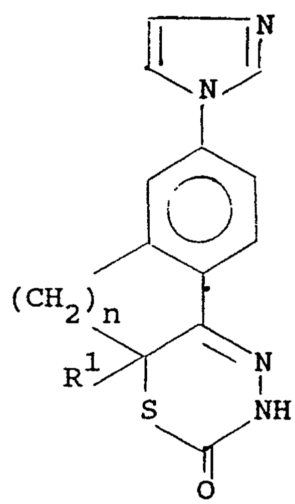

(1)

and pharmaceutically acceptable salts thereof, wherein :
$R^1$ is hydrogen or methyl; and
n is one, and when $R^1$ is hydrogen n can also be two.

Suitably $R^1$ is hydrogen. Suitably $R^1$ is methyl.

In a favoured aspect n is one thus forming a dihydroindenothiadiazinone ring system. In an alternative aspect n is two thus forming a dihydronaphthothiadiazinone ring system.

Specific compounds of this invention include :
7-(1H-imidazol-1-yl)-9,9a-dihydroindeno[1,2-e][1,3,4]-thiadiazin-2(3H)-one,
7-(1H-imidazol-1-yl)-9a-methyl-9,9a-dihydroindeno[1,2-e][1,3,4,]-thiadiazin-2(3H)-one, and
7-(1H-imidazol-1-yl)-9,10-dihydro-10aH-naphtho[1,2-e][1,3,4]-thiadiazin-2(3H)-one,
and pharmaceutically acceptable salts thereof.

This invention covers all tautomeric forms of the compounds of the formula (1) and all optical isomeric forms thereof.

Compounds of the formula (1) may form pharmaceutically acceptable acid-addition salts with either organic or inorganic acids, for example those formed with hydrochloric, hydrobromic, hydriodic, methanesulphonic, sulphuric, maleic, fumaric, succinic, acetic, oxalic, tartaric, citric and lactic acids.

In order to use a compound of the formula (1) or a pharmaceutically acceptable salt thereof for the treatment of mammals including humans it is normally formulated in accordance with standard pharmaceutical practice as a pharmaceutical composition.

Compounds of formula (1) and their pharmaceutically acceptable salts may be administered in standard

manner for the treatment of the indicated diseases, for example orally, parenterally, trans-dermally, rectally, via inhalation or via buccal administration. preferably the compounds of formula (1) and their pharmaceutically acceptable salts are administered parenterally.

Compounds of formula (1) and their pharmaceutically acceptable salts which are active when given orally or via buccal administration can be formulated as syrups, tablets, capsules and lozenges. A syrup formulation will generally consist of a suspension or solution of the compound or salt in a liquid carrier for example, ethanol, glycerine or water with a flavouring or colouring agent. Where the composition is in the form of a tablet, any pharmaceutical carrier routinely used for preparing solid formulations may be used. Examples of such carriers include magnesium stearate, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile aqueous or non-aqueous carrier optionally containing a parenterally acceptable oil, for example polyethylene glycol, polyvinylpyrrolidone, lecithin, arachis oil, or sesame oil.

A typical suppository formulation comprises a compound of formula (1) or a pharmaceutically acceptable salt thereof which is active when administered in this way, with a binding and/or lubricating agent, for example polymeric glycols, gelatins, cocoa-butter or other low melting vegetable waxes or fats.

Typical transdermal formulations comprise of a conventional aqueous or non-aqueous vehicle, for example a cream, ointment, lotion or paste or in the form of a medicated plaster, patch or membrane.

Typical compositions for inhalation are in the form of a solution, suspension or emulsion that may be administered in the form of an aerosol using a conventional propellant such as dichlorodifluoromethane or trichlorofluoromethane.

Preferably the composition is in unit dosage form, for example an ampoule, vial, pre-filled syringe, tablet, capsule or metered aerosol dose.

Each dosage unit for oral administration contains suitably from 0.01 mg/Kg to 3 mg/Kg, and preferably from 0.05 mg/Kg to 1.5 mg/Kg, and each dosage unit for parenteral administration contains suitably from 0.001 mg/Kg to 1 mg/Kg, of a compound of formula (1) or a pharmaceutically acceptable salt thereof calculated as the free base.

The daily dosage regimen for oral administration is suitably about 0.01 mg/Kg to 12 mg/Kg, of a compound of formula (1) or a pharmaceutically acceptable salt thereof calculated as the free base. The daily dosage regimen for parenteral administration is suitably about 0.001 mg/Kg to 4 mg/Kg, for example about 0.01 mg/Kg to 1 mg/Kg, of a compound of the formula (1) or a pharmaceutically acceptable salt thereof calculated as the free base. The active ingredient may be administered as required, for example from 1 to 8 times a day or by infusion, sufficient to increase cardiac output. The compositions of the present invention have positive inotropic activity and vasodilator activity and are of use in the treatment of cardiovascular diseases which can be treated by compounds having either or both of these activities. One such disease condition is congestive heart failure. The compounds of the invention are also bronchodilators and are useful in chronic obstructive lung disease for example asthma and bronchitis. Such conditions can be treated by administration orally, rectally, parenterally or by inhalation. For administration by inhalation dosages are controlled by a valve, are administered as required and for an adult are conveniently in the range 0.1-5.0 mg of a compound of the formula (1) or a pharmaceutically acceptable salt thereof.

The compounds of this invention may be co-administered with other pharmaceutically active compounds, for example in combination, concurrently or sequentially. Conveniently the compounds of this invention and the other active compound or compounds are formulated in a pharmaceutical composition. Examples of compounds which may be included in pharmaceutical compositions with the compounds of the formula (1) are vasodilators for example hydralazine, angiotensin converting enzyme inhibitors for example captopril, anti-anginal agents for example isosorbide nitrate, glyceryl trinitrate and pentaerythritol tetranitrate, anti-arrhythmic agents for example quinidine, procainamide and lignocaine, cardioglycosides for example digoxin and digitoxin, calcium antagonists for example verapamil and nifedipine, diuretics such as thiazides and related compounds for example bendrofluazide, chlorothiazide, chlorothalidone, hydrochlorothiazide, and other diuretics for example frusemide and triamterene, and sedatives for example nitrazepam, flurazepam and diazepam.

In another aspect the present invention provides a process for the preparation of a compound of the formula (1) or a pharmaceutically acceptable salt thereof, which process comprises:

a) for preparing a compound of the formula (1) wherein $R^1$ is hydrogen, reacting a compound of the formula (2) :-

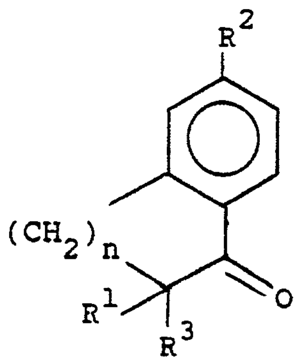

(2)

wherein $R^1$ is hydrogen, $R^2$ is 1-imidazolyl or a precursor thereof, $R^3$ is halo, and n is as hereinbefore defined, with a compound of the formula (3):

$R^4OCSNHNH_2$ (3)

wherein $R^4$ is $C_{1-4}$alkyl; or

b) cyclizing a compound of the formula (4) :

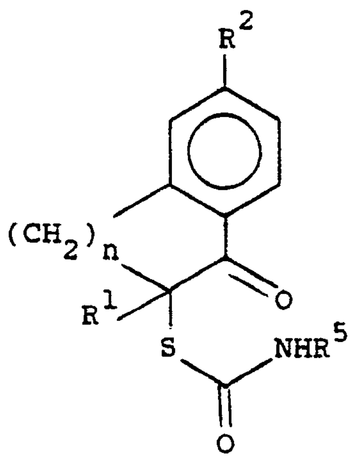

(4)

wherein $R^1$ is hydrogen or methyl and $R^2$ and n are as hereinbefore defined, and $R^5$ is optionally protected amino, in the presence of acid;

and thereafter if necessary :

i) converting a group $R^2$ to 1-imidazolyl,

ii) removing any protecting group,

iii) forming a pharmaceutically acceptable salt.

Suitably the reaction of the compounds of the formulae (2) and (3) is performed in an organic solvent for example a $C_{1-4}$alkanol such as ethanol, or in acetonitrile. The reaction is conveniently performed at an elevated temperature for example under reflux conditions. Suitably $R^3$ is bromo or chloro, preferably bromo. Suitably $R^4$ is methyl.

Suitably the cyclization of a compound of the formula (4) is performed in an aqueous inorganic acid, for example hydrochloric acid, or in an organic solvent containing an aqueous inorganic acid, for example in dimethylformamide or a $C_{1-6}$alkanol, such as ethanol in admixture with hydrochloric acid. The cyclization is conveniently performed at an elevated temperature for example 60° C to 140° C, preferably at reflux temperature for convenience.

The cyclization may be performed on a compound of the formula (4) wherein $R^5$ is amino, or a protected variant of the compound of the formula (4), for example protected on the hydrazine function by an acid-labile protecting group for example isopropylidene or benzylidene i.e. $R^5$ is $-N=C(CH_3)_2$ or $-N=CHC_6H_5$.

An example of $R^2$ being a precursor of 1-imidazolyl is when $R^2$ is a halo group, preferably fluoro, which may react with imidazole at an elevated temperature in the absence of a solvent or in an organic solvent such as dimethylsulphoxide, dimethylformamide or N-methylpyrrolidone optionally in the presence of a base such as an alkali metal carbonate.

Pharmaceutically acceptable salts of the compounds of the formula (1) may be prepared in conventional manner, for example acid addition salts may be prepared by treating the compounds of the formula (1) with the appropriate acid in a $C_{1-4}$alkanol, or they may be prepared by the use of an ion-exchange resin to form the desired salt directly from the free base or via a different acid addition salt.

The compounds of the formula (4) may be conveniently prepared by reacting a compound of the formula (2) wherein $R^1$ is hydrogen or methyl, and $R^2$, $R^3$ and n are as hereinbefore defined, with a compound of the formula (5):

$M^{\oplus}\ {}^{\ominus}O\text{-}CS\text{-}NHR^5$ (5)

wherein $R^5$ is as hereinbefore defined and $M^+$ is a counter-ion, for example an alkali metal ion such as potassium or sodium or is an ammonium ion.

Suitably the reaction of the compounds of the formulae (2) and (5) is performed in an organic solvent such as a $C_{1-4}$alkanol, dimethylformamide or acetonitrile. The reaction is conveniently performed at a non-extreme temperature for example between -10° C and 80° C, preferably between 0° and 30° C.

The compound of the formula (4) need not be isolated but may be cyclized in situ in the presence of acid as hereinbefore described.

If it is desired to prepare a protected compound of the formula (4) then the compound of the formula (5) may be in protected form, for example as the isopropylidene. In an alternative the compound of the formula (4) may be protected, if desired, after the reaction of the compounds of the formulae (2) and (5).

The compounds of the formula (2) may be conveniently prepared by halogenating a compound of the formula (6) :-

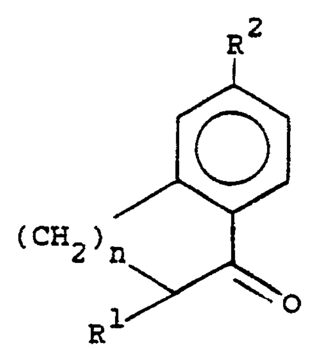

(6)

wherein $R^1$ is hydrogen or methyl, and $R^2$ and n are as hereinbefore defined. Suitably to prepare compounds wherein $R^3$ is bromo, the reaction is performed in a chlorinated organic solvent, for example chloroform with a solution of bromine. The reaction is conveniently performed at a non-extreme temperature such as between -20° and 60° C, preferably between 0° and 30° C. Preferably to prepare compounds wherein $R^2$ is 1-imidazolyl and $R^3$ is bromo, the reaction is performed in a solution of bromine and hydrogen bromide in acetic acid at a non-extreme temperature such as between -20° and 80° C, preferably between 30° and 70° C.

The compounds of the formula (6) wherein $R^2$ is 1-imidazolyl may be prepared from the compounds of the formula (6) wherein $R^2$ is a precursor to 1-imidazolyl such as fluoro, by reaction with imidazole in an analogous manner to that hereinbefore described for the preparation of compounds of the formula (1).

The compounds of the formula (6) wherein $R^2$ is fluoro are known or preparable in conventional manner from Ep-A-145,236.

The following biological test methods, data and Example serve to illustrate this invention.

Cardiac Stimulant Activity - In vivo (Anaesthetised Cats)

In anaesthetised cats pretreated with a ganglion blocker (pempidine) and propranolol, the compounds of the Examples caused sustained increases in left ventricular dp/dt max (this is an index of left ventricular

contractility) when administered intravenously. The dose to increase left ventricular dp/dt max by 50% is given as the $ED_{50}$. The compound of Example 1 gave an $ED_{50}$ (micromol/kg) value of 0.09 and displayed a short duration of activity. In comparison amrinone gave a value of 5.6.

A compound with a rapid onset and short duration of activity is particularly useful for intra venous (i.v.) infusion, for example for acute i.v. use in the treatment of congestive heart failure or status asthmaticus, since i.v. infusion of such a compound has the following advantages :

i) the pharmacological effect is rapid when the infusion is commenced,
ii) the titration of the dose to suit the individual patient is facilitated, and
iii) the pharmacological effect is not sustained when infusion is halted.

Inhibition of phosphodiesterases

Three peaks of cyclic nucleotide phosphodiesterase activity [PDE (peak I), PDE (peak II) and PDE (Peak III)] from cat heart were separated by chromatography on DEAE-Sepharose CL-6B (Diethylaminoethyl Cellulose with a bead size of 45-165 microns). Sepharose is a registered trademark of Pharmacia Fine Chemicals Inc. The high-speed supernatant from a cat heart homogenate (2 g) tissue in 20 ml 20 mM PIPES (Piperazine-N-N'-bis[2-ethanesulfonic acid]), 50 mM Na acetate, pH 6.5) was applied to a 15 × 1.5 cm column of DEAE-Sepharose equilibrated with the homogenisation buffer. The PDE activities were eluted with a gradient of 0.05-1 M Na acetate in 20 mM PIPES. There were three major peaks which had the following characteristics:

PDE (Peak I) - eluted at 0.15 M Na acetate

| Substrate | 50 μg/ml calmodulin (+ = added) | Km (μM) | Relative $V_{max}$ |
|---|---|---|---|
| cyclic AMP | - | 0.5 | 1 |
| cyclic GMP | - | 1.8 | 1.1 |
| cyclic AMP | + | 0.7 | 6.3 |
| cyclic GMP | + | 1.4 | 7.2 |

PDE (Peak II) - eluted at 0.3 M Na acetate

| Substrate | Km(μM) | Relative $V_{max}$ |
|---|---|---|
| cyclic AMP | 6 | 1 |
| cyclic GMP | 28 | 0.2 |

PDE (Peak III) - eluted at 0.5 M Na acetate

| Substrate | Km (μM) | Relative $V_{max}$ |
|---|---|---|
| cyclic AMP | 0.6 | 1 |
| cyclic GMP | 2.9 | 0.4 |

PDE (Peak I) has high affinity for cyclic AMP and cyclic GMP and is characterised by an activation by $Ca^{2+}$/calmodulin complex.

PDE (Peak II) demonstrates relatively low affinities for both cyclic AMP and cyclic GMP and is not affected by $Ca^{2+}$/calmodulin complex.

PDE (Peak III) has high affinity for cyclic AMP. It can also hydrolyse cyclic GMP though the preferred substrate is cyclic AMP. This activity is also insensitive to $Ca^{2+}$/calmodulin activation.

Enzyme assay

The enzyme was assayed by incubation at 37° for 4-30 min in 50 mM Tris, 5 mM $MgCl_2$, pH 7.5 with [3-H] cyclic nucleotide ($4 \times 10^5$ disintegrations $min^{-1}$) and [14-C] nucleotide 5′ monophosphate ($3 \times 10^3$ disintegrations $min^{-1}$). The assay was stopped by boiling, and the [3-H] 5′monophosphate product

separated from substrate on boronate columns (Davis, C.W. And Daly, J.W. (1979) J. Cyclic Nucleotide Res., 5, 65-74). The reaction mixture was diluted with 0.5 ml 100 mM HEPES (N-2-Hydroxyethyl-piperazine-N'-2-ethanesulfonic acid), 100 mM NaCl, pH 8.5, and applied to the column. The column was extensively washed with the same buffer, and the 5' nucleotide eluted with 6 ml 0.25 M acetic acid. The recovery of product as judged by [14-C] recovery was approximately 80%. All assays were linear with time of incubation and concentration of enzyme over the range used in these experiments.

Calculation of $IC_{50}$ values

$IC_{50}$ values (the concentration of inhibitor required for 50% inhibition of activity) were obtained for PDE (Peak III) by incubation of the enzyme at 1 µM cyclic AMP, and a range of inhibitor concentrations from 0.1 × $IC_{50}$ to 100 × $IC_{50}$.

| Compound of Example | $IC_{50} \times 10^{-6}M$ |
|---|---|
| 1 | 0.54 |
| Amrinone | 51.8 |
| Milrinone | 2.2 |

Example 1

7-(1H-Imidazol-1-yl)-9,9a-dihydroindeno[1,2-e][1,3,4]-thiadiazin-2(3H)-one

a) A stirred melt of 5-fluoro-1-indanone (15 g) and imidazole (30 g) was heated at 160-170° C for 2 hours under nitrogen. The melt was allowed to cool to about 80° C then it was digested with hot ethyl acetate (500 ml) and the filtered digest was cooled, washed with water, and extracted with dilute hydrochloric acid. The extract was washed with dichloromethane, then treated with potassium carbonate to pH 8 to give a crude product, 8.78 g. Recrystallisation from water gave a solid 6.34 g, m.p. 147.5-149° C which was recrystallised from toluene to give 5-(1H-imidazol-1-yl)-1-indanone, m.p. 148-150° C, in 78% recovery.

b) Bromine (0.54 ml) was added dropwise to a stirred solution of 5-(1H-imidazol-1-yl)-1-indanone (2 g) in acetic acid (20 ml) containing hydrogen bromide (1.82 ml of a 45% w/v solution in acetic acid) at 70° C. After 30 minutes the solution was added to cold water (100 ml) and the solution was cooled in ice while potassium carbonate was added to pH 6. The aqueous solution was decanted from a gum, extracted with dichloromethane (60 ml), the extract combined with the gum and the resultant solution was washed with brine, dried over sodium sulphate, and evaporated under reduced pressure at room temperature to a volume of 10-20 ml. This solution contained 2-bromo-5-(1H-imidazol-1-yl)-1-indanone.

To the above solution was added potassium thiocarbazate (1.95 g), potassium bicarbonate (1 g) and dry dimethylformamide (DMF) (20 ml). Residual dichloromethane was removed by evaporation on a rotary evaporator under reduced pressure at 30° C to leave a suspension in DMF, which was rotated at 30° C for a further one hour. Water (80 ml) was then added, followed by concentrated hydrochloric acid to pH 2. The solution was heated on a steam bath for 5 minutes, then cooled and potassium bicarbonate was added to pH 7-8 to give a crude product, 1.93 g, m.p. 220° C dec. Recrystallisation from aqueous ethanol afforded the pure title compound, 1.1 g, m.p. 235-237° C dec.

Example 2

7-(1H-Imidazol-1-yl)-9,9a-dihydroindeno[1,2-e][1,3,4,]-thiadiazin-2(3H)-one

Bromine (0.14 ml) in a little acetic acid was added to a stirred solution of 5-(1H-imidazol-1-yl)-1-indanone (0.5 g) in acetic acid (5 ml) containing hydrogen bromide (0.48 ml of a 45% w/v solution in acetic acid) at 70 °C.

After 30 minutes the mixture was allowed to cool and methoxythiocarbonylhydrazine (0.4 g) was added and the stirred mixture was heated under reflux for 1 hour. Evaporation left a gum which was dissolved in water (25 ml) and sodium bicarbonate was added to pH 6-7 to give a crude product, 0.55 g, m.p. 160-185 °C dec, thin layer chromatography analysis of which indicated the presence of the same product as obtained in Example 1 (b) above.

Example 3

7-(1H-Imidazol-1-yl)-9a-methyl-9,9a-dihydroindeno[1,2-e][1,3,4]thiadiazin-2(3H)-one

a) A stirred melt of 5-fluoro-2-methyl-1-indanone (4.9 g) and imidazole (9.8 g) was heated under nitrogen at 175 °C for 2 hours. The warm melt was diluted with ethyl acetate (100 ml), the resulting solution extracted with dilute hydrochloric acid, and the acidic extract treated with potassium carbonate to pH 6. Extraction of the mixture with ethyl acetate and with dichloromethane failed to dissolve all of the solids and the mixture was filtered to remove a solid by-product, 5-(1H-imidazol-1-yl)-2-methyl-1H-inden-1-one, 0.92 g, m.p. 215-217 °C (from toluene). The combined aqueous and organic solutions were evaporated to leave an aqueous mixture which deposited a sticky solid; further product was obtained by ethyl acetate extraction. The combined crude product (5.36 g) was purified by medium pressure column chromatography (silica gel, chloroform) to give 5-(1H-imidazol-1-yl)-2-methyl-1-indanone, 3 g (47%), m.p. 103-106 °C, which was used directly in the next stage.

b) Following a procedure similar to that of Example 1(b), 5-(1H-imidazol-1-yl)-2-methyl-1-indanone (0.75 g) gave the crude title compound, 0.68 g. Re-precipitation of this solid from water (made acidic (pH 2) with 2 Normal HCl) by the addition of 2 Normal $NH_4OH$ to ca pH 6 gave the title compound, 0.56 g (56%), m.p. ca 253-255 °C dec. δ(DMSO-$d_6$) : 1.53 (3H, s); 3.34 (2H, s); 7.17 (1H, s), 7.7-7.86 (4H, m) and 8.38 (1H, s); 11.7 (1H, s).

Example 4

A pharmaceutical composition for parenteral administration is prepared by dissolving the title compound of Example 1 (0.02 g) in polyethylene glycol 300 (25 ml) with heating. This solution is then diluted with water for injections Ph. Eur. (to 100 ml). The solution is then sterilised by filtration through a 0.22 micron membrane filter and sealed in sterile containers.

Compositions containing the compound of Example 1 (0.04 g) in polyethylene glycol 300 are prepared in analogous manner.

Example 5

Pharmaceutical compositions for oral administration are prepared by combining the following :

| | | % w/w | |
|---|---|---|---|
| 7-(1H-Imidazol-1-yl)-9,9a-dihydroindeno[1,2-e][1,3,4]-thiadiazin-2(3H)-one | 0.5 | 3.0 | 7.14 |
| 2% w/w Soya lecithin in soya bean oil | 90.45 | 88.2 | 84.41 |
| Hydrogenated vegetable shortening and beeswax | 9.05 | 8.8 | 8.45 |

The formulations are then filled into individual soft gelatin capsules.

**Claims**

**Claims for the following Contracting States: BE, CH, DE, FR, GB, IT, LI, LU, NL, SE**

1. A compound of the formula (1) :-

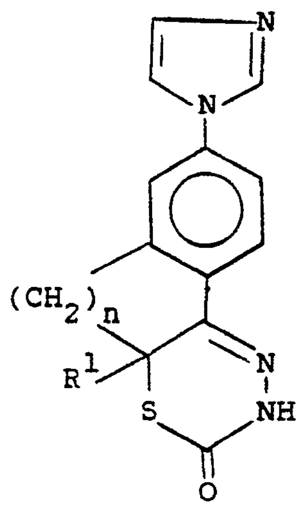

(1)

or a pharmaceutically acceptable salt thereof, wherein :

$R^1$ is hydrogen or methyl; and

n is one, and when $R^1$ is hydrogen n can also be two.

2. A compound according to claim 1 wherein n is one.

3. A compound according to claim 1 or 2 wherein $R^1$ is hydrogen.

4. A compound according to claim 1 which is :

7-(1H-imidazol-1-yl)-9,9a-dihydroindeno[1,2-e][1,3,4]-thiadiazin-2(3H)-one,

7-(1H-imidazol-1-yl)-9a-methyl-9,9a-dihydroindeno[1,2-e][1,3,4]-thiadiazin-2(3H)-one, or

7-(1H-imidazol-1-yl)-9,10-dihydro-10aH-naphtho[1,2-e][1,3,4]-thiadiazin-2(3H)-one,

or a pharmaceutically acceptable salt thereof.

5. A pharmaceutical composition which comprises a compound according to any of claims 1 to 4 and a pharmaceutically acceptable carrier.

6. A process for the preparation of a compound of the formula (1) as defined in claim 1 or a pharmaceutically acceptable salt thereof, which process comprises:
a) for preparing a compound of the formula (1) wherein $R^1$ is hydrogen, reacting a compound of the formula (2) :-

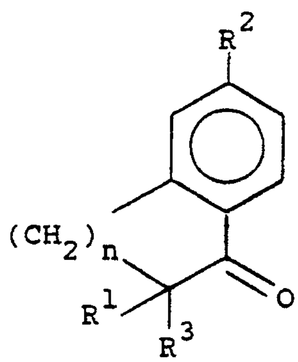
(2)

wherein $R^1$ is hydrogen, $R^2$ is 1-imidazolyl or a precursor thereof, $R^3$ is halo, and n is as defined in claim 1, with a compound of the formula (3):

$R^4OCSNHNH_2$ (3)

wherein $R^4$ is $C_{1-4}$alkyl; or
b) cyclizing a compound of the formula (4) :

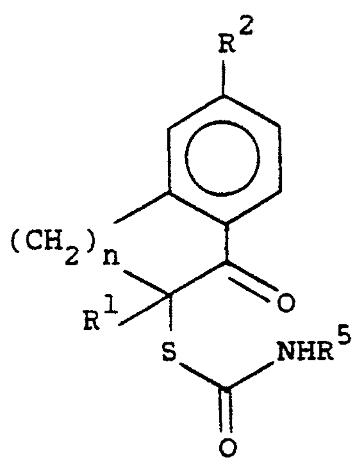
(4)

wherein $R^1$ is hydrogen or methyl and $R^2$ and n are as hereinbefore defined, and $R^5$ is optionally protected amino, in the presence of acid;
and thereafter if necessary :
i) converting a group $R^2$ to 1-imidazolyl,
ii) removing any protecting group,
iii) forming a pharmaceutically acceptable salt.

7. A process according to claim 6 wherein the compound of the formula (4) is prepared by reaction of a compound of the formula (2) :

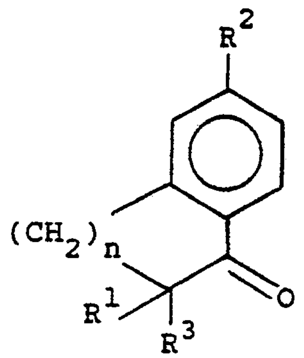

(2)

wherein $R^1$ is hydrogen or methyl, and $R^2$, $R^3$ and n are as defined in claim 6, with a compound of the formula (5) :

$M^{\oplus}\ {}^{\ominus}O\text{-}CS\text{-}NHR^5$ (5)

wherein $R^5$ is optionally protected amino and $M^+$ is a counter-ion.

8. A compound according to any one of claims 1 to 4 for use as a medicament.

9. A compound according to any one of claims 1 to 4 for use as an inotropic agent.

10. A compound of the formula (4) :

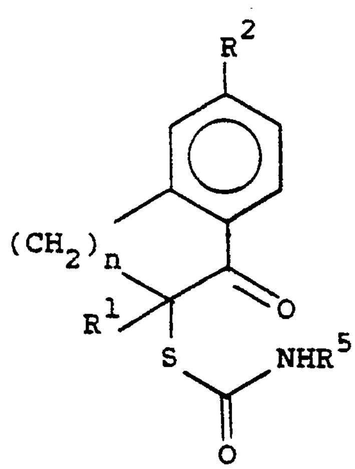

(4)

wherein $R^1$ and n are as defined in claim 1,

$R^2$ is 1-imidazolyl and

$R^5$ is optionally protected amino.

11. The use of a compound of the formula (1) as defined in claim 1 or a pharmaceutically acceptable salt thereof in the manufacture of a medicament for the treatment of congestive heart failure.

**Claims for the following Contracting States: AT, ES, GR**

1. A process for preparing a compound of the formula (1) :-

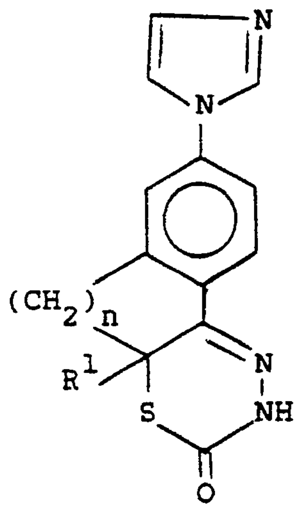

(1)

or a pharmaceutically acceptable salt thereof, wherein :

$R^1$ is hydrogen or methyl; and

n is one, and when $R^1$ is hydrogen n can also be two;

which process comprises:

a) for preparing a compound of the formula (1) wherein $R^1$ is hydrogen, reacting a compound of the formula (2) :-

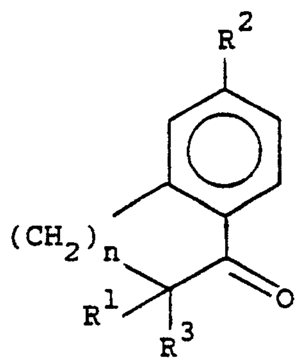

(2)

wherein $R^1$ is hydrogen, $R^2$ is 1-imidazolyl or a precursor thereof, $R^3$ is halo, and n is as hereinbefore defined, with a compound of the formula (3):

$R^4OCSNHNH_2$ (3)

wherein $R^4$ is $C_{1-4}$alkyl; or

b) cyclizing a compound of the formula (4) :

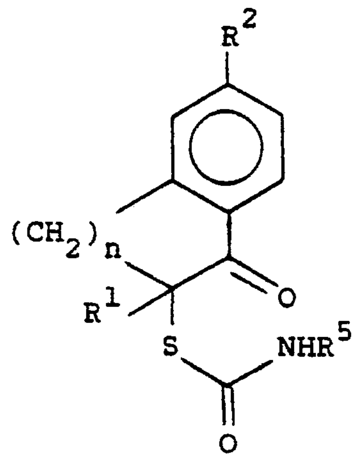

(4)

wherein $R^1$ is hydrogen or methyl and $R^2$ and n are as hereinbefore defined, and $R^5$ is optionally protected amino, in the presence of acid;

and thereafter if necessary :

i) converting a group $R^2$ to 1-imidazolyl,

ii) removing any protecting group,

iii) forming a pharmaceutically acceptable salt.

2. A process according to claim 1 for preparing a compound wherein n is one.

3. A process according to claim 1 or 2 for preparing a compound where in $R^1$ is hydrogen.

4. A process according to claim 1 for preparing a compound which is :

7-(1H-imidazol-1-yl)-9,9a-dihydroindeno[1,2-e][1,3,4]-thiadiazin-2(3H)-one,

7-(1H-imidazol-1-yl)-9a-methyl-9,9a-dihydroindeno[1,2-e][1,3,4]-thiadiazin-2(3H)-one, or

7-(1H-imidazol-1-yl)-9,10-dihydro-10aH-naphtho[1,2-e][1,3,4]-thiadiazin-2(3H)-one,

or a pharmaceutically acceptable salt thereof.

5. A process for preparing a pharmaceutical composition which comprises bringing into association a compound of the formula (1) or a pharmaceutically acceptable salt thereof as defined in claim 1 and a pharmaceutically acceptable carrier.

6. A process according to claim 1 wherein the compound of the formula (4) is prepared by reaction of a compound of the formula (2) :

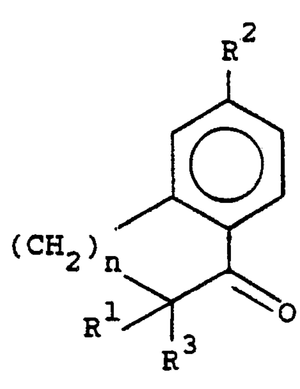

(2)

wherein $R^1$ is hydrogen or methyl and $R^2$, $R^3$ and n are as defined in claim 1, with a compound of the formula (5) :

$M^{\oplus}\ {}^{\ominus}O\text{-}CS\text{-}NHR^5$ (5)

wherein $R^5$ is optionally protected amino and $M^+$ is a counter-ion.

**7.** A process according to claim 6 which comprises reacting 2-bromo-5-(1H-imidazol-1-yl)-1-indanone with potassium thiocarbazate, and subsequently treating with acid to give 7-(1H-imidazol-1-yl)-9,9a-dihydroindeno[1,2-e][1,3,4]thiadiazin-2(3H)-one.

**8.** A process for preparing a compound of the formula (4) :

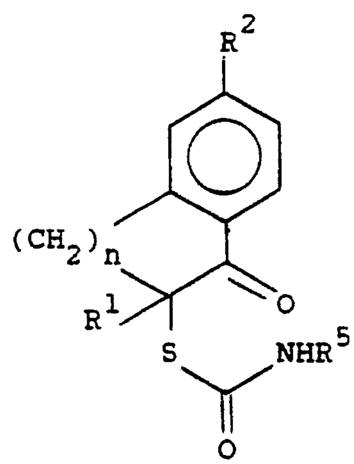

(4)

wherein $R^1$ and n are as defined in claim 1,

$R^2$ is 1-imidazolyl and

$R^5$ is optionally protected amino,

which comprises reacting a compound of formula (2) :

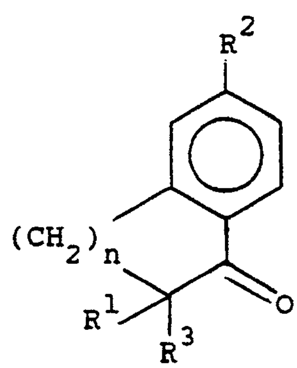

(2)

wherein $R^1$, $R^2$ and n are as hereinbefore defined and $R^3$ is halo, with a compound of the formula (5) :

$M^{\oplus}\ {}^{\ominus}O\text{-}CS\text{-}NHR^5$ (5)

wherein $R^5$ is optionally protected amino and $M^+$ is a counter-ion.

**9.** The use of a compound of the formula (1) as defined in claim 1 or a pharmaceutically acceptable salt thereof in the manufacture of a medicament for the treatment of congestive heart failure.

**Revendications**

**Revendications pour les Etats contractants suivants: BE, CH, DE, FR, GB, IT, LI, LU, NL, SE**

**1.** Composé de formule (1) :

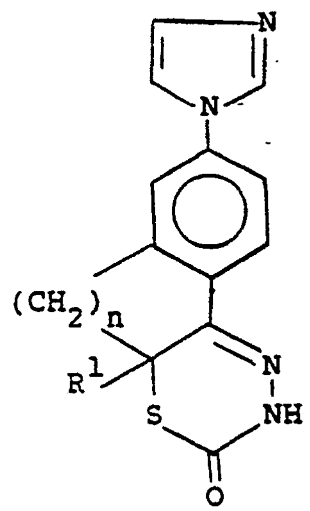

(1)

ou un sel pharmaceutiquement acceptable de celui-ci, dans lequel :
$R^1$ représente un atome d'hydrogène ou un groupe méthyle ; et
n est égal à un, et lorsque $R^1$ représente un atome d'hydrogène, n peut également être égal à deux.

**2.** Composé selon la revendication 1, dans lequel n est égal à un.

**3.** Composé selon la revendication 1 ou 2, dans lequel $R^1$ représente un atome d'hydrogène.

**4.** Composé selon la revendication 1, qui est
la 7-(1H-imidazol-1-yl)-9,9a-dihydroindéno[1,2-e][1,3,4]-thiadiazin-2(3H)-one,
la 7-(1H-imidazol-1-yl)-9,9a-dihydroindéno[1,2-dihydroindéno[1,2-e][1,3,4]-thiadiazin-2(3H)-one, ou
la 7-(1H-imidazol-1-yl)-9,10-dihydro-10aH-naphto[1,2-e][1,3,4]-thiadiazin-2(3H)-one,
ou un sel pharmaceutiquement acceptable de celle-ci.

**5.** Composition pharmaceutique, comprenant un composé selon l'une quelconque des revendications 1 à 4, et un véhicule pharmaceutiquement acceptable.

**6.** Procédé de préparation d'un composé de formule (1) selon la revendication 1, ou d'un sel pharmaceutiquement acceptable de celui-ci, selon lequel :
a) pour préparer un composé de formule (1) dans laquelle $R^1$ représente un atome d'hydrogène, on fait réagir un composé de formule (2) :

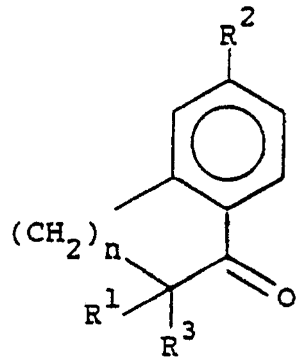

(2)

dans laquelle $R^1$ représente un atome d'hydrogène, et $R^2$ représente un groupe 1-imidazolyle ou un précurseur de celui-ci, $R^3$ représente un groupe halo, et n est tel que défini dans la revendication 1, avec un composé de formule (3) :

$R^4OCSNHNH_2$ (3)

dans laquelle $R^4$ représente un groupe alkyle en $C_1$-$C_4$ ; ou
b) on cyclise un composé de formule (4) :

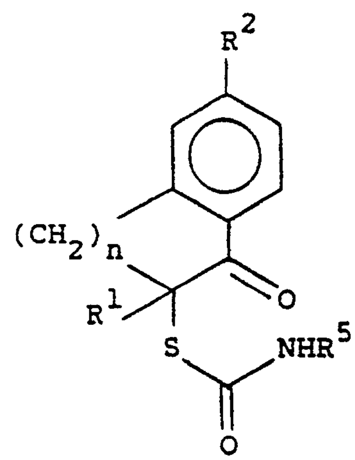

(4)

dans laquelle $R^1$ représente un atome d'hydrogène ou un groupe méthyle, et $R^2$ ainsi que n sont tels que définis ci-dessus, et $R^5$ est un groupe amino éventuellement protégé, en présence d'un acide ; et ensuite, si cela est nécessaire :
i) on convertit un groupe $R^2$ en un groupe 1-imidazolyle,
ii) on élimine les groupes protecteurs,
iii) on forme un sel pharmaceutiquement acceptable.

7. Procédé selon la revendication 6, dans lequel le composé de formule (4) est préparé en faisant réagir un composé de formule (2) :

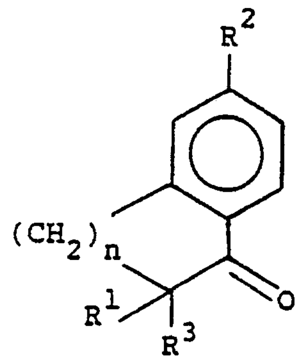

(2)

dans laquelle $R^1$ représente un atome d'hydrogène ou un groupe méthyle, et $R^2$, $R^3$ ainsi que n sont tels que définis dans la revendication 6, avec un composé de formule (5) :

$M^{(+)}\,{}^{(-)}O\text{-}CS\text{-}NHR^5$ (5)

dans laquelle $R^5$ représente un groupe amino éventuellement protégé, et $M^+$ est un contre-ion.

**8.** Composé selon l'une quelconque des revendications 1 à 4, destiné à être employé comme médicament.

**9.** Composé selon l'une quelconque des revendications 1 à 4, destiné à être employé comme agent inotrope.

**10.** Composé de formule (4) :

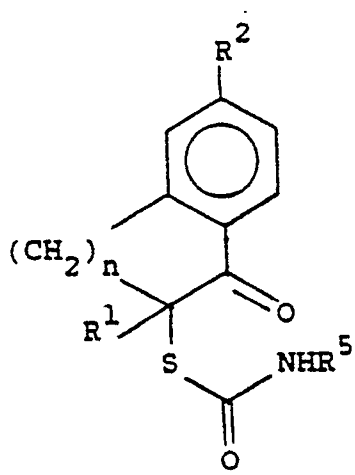

(4)

dans laquelle $R^1$ et n sont tels que définis dans la revendication 1,
$R^2$ représente un groupe 1-imidazolyle, et
$R^5$ représente un groupe amino éventuellement protégé.

**11.** Utilisation d'un composé de formule (1) selon la revendication 1 ou d'un sel pharmaceutiquement acceptable de celui-ci, pour fabriquer un médicament pour le traitement de l'insuffisance cardiaque.

**Revendications pour les Etats contractants suivants: AT, ES, GR**

**1.** Procédé de préparation d'un composé de formule (1) :

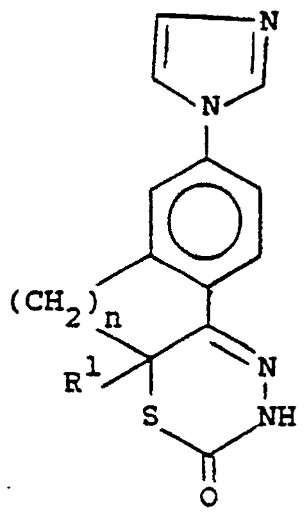

(1)

ou d'un sel pharmaceutiquement acceptable de celui-ci, dans lequel :
$R^1$ représente un atome d'hydrogène ou un groupe méthyle ; et
n est égal à un, et lorsque $R^1$ représente un atome d'hydrogène, n peut également être égal à deux ;
selon lequel :

a) pour préparer un composé de formule (1) dans laquelle $R^1$ représente un atome d'hydrogène, on fait réagir un composé de formule (2) :

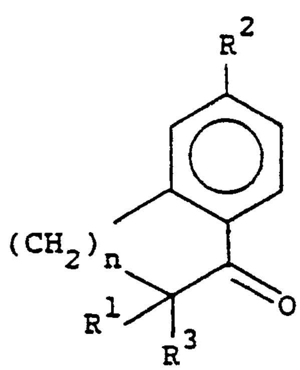

(2)

dans laquelle $R^1$ représente un atome d'hydrogène, $R^2$ représente un groupe 1-imidazolyle ou un précurseur de celui-ci, $R^3$ représente un groupe halo, et n est tel que défini ci-dessus, avec un composé de formule (3) :

$R^4OCSNHNH_2$ (3)

dans laquelle $R^4$ représente un groupe alkyle en $C_1$-$C_4$ ; ou
b) on cyclise un composé de formule (4) :

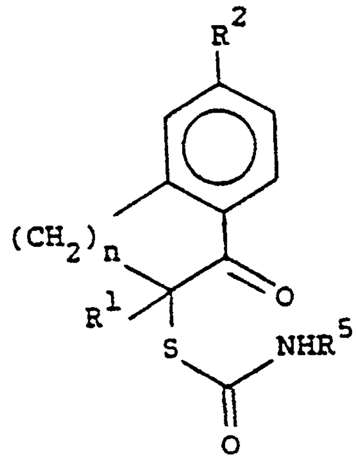

(4)

dans laquelle $R^1$ représente un atome d'hydrogène ou un groupe méthyle, et $R^2$ ainsi que n sont tels que définis ci-dessus, et $R^5$ représente un groupe amino éventuellement protégé, en présence d'un acide ; et ensuite, si cela est nécessaire :
i) on convertit un groupe $R^2$ en un groupe 1-imidazolyle,
ii) on élimine les groupes protecteurs,
iii) on forme un sel pharmaceutiquement acceptable.

**2.** Procédé selon la revendication 1, de préparation d'un composé dans lequel n est égal à un.

**3.** Procédé selon la revendication 1 ou 2, pour préparer un composé dans lequel $R^1$ représente un atome d'hydrogène.

**4.** Procédé selon la revendication 1, pour préparer un composé qui est
la 7-(1H-imidazol-1-yl)-9,9a-dihydroindéno[1,2-e][1,3,4]-thiadiazin-2(3H)-one,
la 7-(1H-imidazol-1-yl)-9a-méthyl-9,9a-dihydroindéno[1,2-e][1,3,4]-thiadiazin-2(3H)-one, ou
la 7-(1H-imidazol-1-yl)-9,10-dihydro-10aH-naphto[1,2-e][1,3,4]-thiadiazin-2(3H)-one,
ou un sel pharmaceutiquement acceptable de celle-ci.

**5.** Procédé de préparation d'une composition pharmaceutique, selon lequel on associe un composé de formule (1) ou un sel pharmaceutiquement acceptable de celui-ci tel que défini dans la revendication 1, et un véhicule pharmaceutiquement acceptable.

**6.** Procédé selon la revendication 1, dans lequel le composé de formule (4) est préparé en faisant réagir un composé de formule (2) :

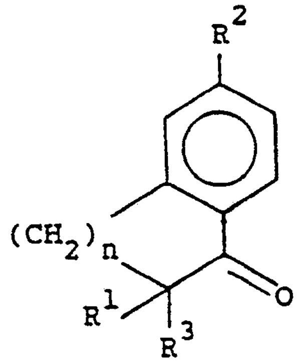

(2)

dans laquelle $R^1$ représente un atome d'hydrogène ou un groupe méthyle, et $R^2$, $R^3$, et n sont tels que définis dans la revendication 1, avec un composé de formule (5) :

$M^{(+)}\,{}^{(-)}O\text{-}CS\text{-}NHR^5$ (5)

dans laquelle $R^5$ représente un groupe amino éventuellement protégé, et $M^+$ est un contre-ion.

7. Procédé selon la revendication 6, dans lequel on fait réagir la 2-bromo-5-(1H-imidazol-1-yl)-1-indanone avec le thiocarbazate de potassium, et on effectue ensuite un traitement avec un acide pour obtenir la 7-(1H-imidazol-1-yl)-9,9a-dihydroindéno-[1,2-e][1,3,4]thiadiazin-2(3H)-one.

8. Procédé de préparation d'un composé de formule (4) :

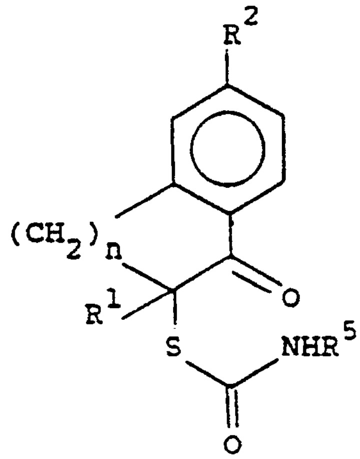

(4)

dans laquelle $R^1$ et n sont tels que définis dans la revendication 1,
$R^2$ représente un groupe 1-imidazolyle, et
$R^5$ représente un groupe amino éventuellement protégé,
selon lequel on fait réagir un composé de formule (2) :

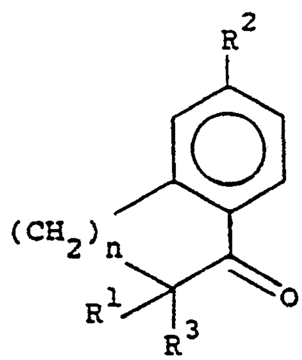

(2)

dans laquelle $R^1$, $R^2$ et n sont tels que définis ci-dessus, et $R^3$ représente un groupe halo, avec un composé de formule (5) :

$M^{(+)}\,{}^{(-)}O\text{-}CS\text{-}NHR^5$ (5)

dans laquelle $R^5$ représente un groupe amino éventuellement protégé, et $M^+$ est un contre-ion.

9. Utilisation d'un composé de formule (1) selon la revendication 1 ou d'un sel pharmaceutiquement acceptable de celui-ci, pour fabriquer un médicament pour le traitement de l'insuffisance cardiaque.

**Patentansprüche**

**Patentansprüche für folgende Vertragsstaaten: BE, CH, DE, FR, GB, IT, LI, LU, NL, SE**

1. Verbindung der Formel (1)

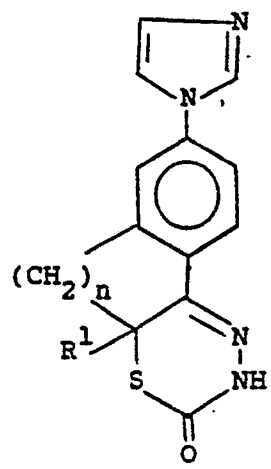

(1)

oder ein pharmazeutisch verträgliches Salz davon, wobei:
$R^1$ ein Wasserstoffatom oder eine Methylgruppe bedeutet; und
n eins ist und wenn $R^1$ ein Wasserstoffatom ist, n auch zwei sein kann.

2. Verbindung nach Anspruch 1, wobei n eins ist.

3. Verbindung nach Anspruch 1 oder 2, wobei $R^1$ ein Wasserstoffatom bedeutet.

4. Verbindung nach Anspruch 1, nämlich:

7-(1H-Imidazol-1-yl)-9,9a-dihydroindeno[1,2-e]-[1,3,4]-thiadiazin-2(3H)-on,

7-(1H-Imidazol-1-yl)-9a-methyl-9,9a-dihydroindeno-[1,2-e]-[1,3,4]-thiadiazin-2(3H)-on oder

7-(1H-Imidazol-1-yl)-9,10-dihydro-10aH-naphtho[1,2-e]-[1,3,4]-thiadiazin-2(3H)-on,

oder ein pharmazeutisch verträgliches Salz davon.

5. Arzneimittel, umfassend eine Verbindung nach einem der Ansprüche 1 bis 4 und einen pharmazeutisch verträglichen Träger.

6. Verfahren zur Herstellung einer Verbindung der Formel (1) gemäß Anspruch 1, oder eines pharmazeutisch verträglichen Salzes davon, welches Verfahren umfaßt:
a) zur Herstellung einer Verbindung der Formel (1), in der $R^1$ ein Wasserstoffatom bedeutet, Umsetzung einer Verbindung der Formel (2)

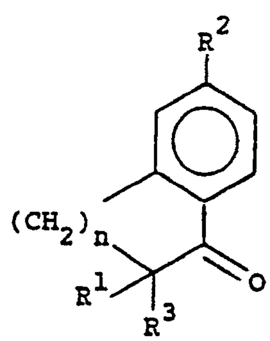

(2)

in der $R^1$ ein Wasserstoffatom bedeutet, $R^2$ eine 1-Imidazolylgruppe oder eine Vorstufe davon darstellt, $R^3$ ein Halogenatom bedeutet und n wie in Anspruch 1 definiert ist, mit einer Verbindung der Formel (3)

$R^4OCSNHNH_2$ (3)

in der $R^4$ einen $C_{1-4}$-Alkylrest darstellt; oder
b) Cyclisierung einer Verbindung der Formel (4)

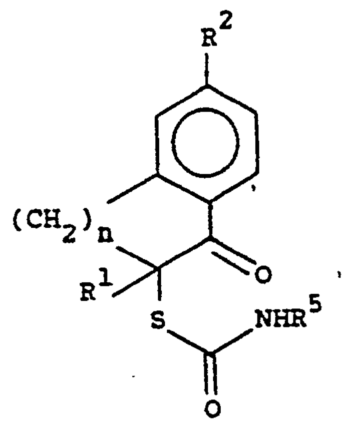

(4)

in der $R^1$ ein Wasserstoffatom oder eine Methylgruppe bedeutet und $R^2$ und n wie vorstehend definiert sind, und $R^5$ eine gegebenenfalls geschützte Aminogruppe darstellt, in Gegenwart einer Säure;

und danach falls erforderlich:
i) Umwandlung des Restes $R^2$ in die 1-Imidazolylgruppe,
ii) Entfernung irgendwelcher Schutzgruppen,
iii) Herstellung eines pharmazeutisch verträglichen Salzes.

7. Verfahren nach Anspruch 6, wobei die Verbindung der Formel (4) durch Umsetzung einer Verbindung der Formel (2)

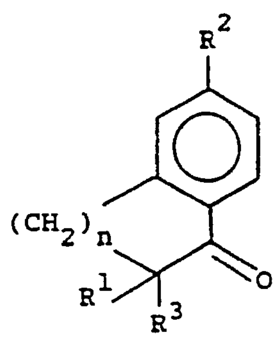

(2)

in der $R^1$ ein Wasserstoffatom oder eine Methylgruppe bedeutet, und $R^2$, $R^3$ und n wie in Anspruch 6 definiert sind, mit einer Verbindung der Formel (5)

$M^{\oplus}\,{}^{\ominus}O\text{-}CS\text{-}NHR^5$ (5)

in der $R^5$ eine gegebenenfalls geschützte Aminogruppe bedeutet, und $M^+$ ein Gegenion darstellt, hergestellt wird.

8. Verbindung nach einem der Ansprüche 1 bis 4 zur Verwendung als Arzneistoff.

9. Verbindung nach einem der Ansprüche 1 bis 4 zur Verwendung als inotroper Wirkstoff.

10. Verbindung der Formel (4)

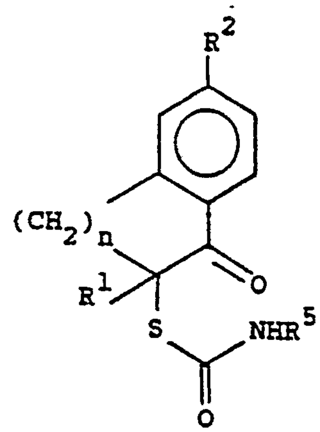

(4)

in der $R^1$ und n wie in Anspruch 1 definiert sind,
$R^2$ eine 1-Imidazolylgruppe bedeutet, und
$R^5$ eine gegebenenfalls geschützte Aminogruppe darstellt.

11. Verwendung einer Verbindung der Formel (1) nach Anspruch 1 oder eines pharmazeutisch verträglichen Salzes davon zur Herstellung eines Arzneimittels zur Behandlung von Stauungsherzversagen.

**Patentansprüche für folgende Vertragsstaaten: AT, ES, GR**

1. Verfahren zur Herstellung einer Verbindung der Formel (1)

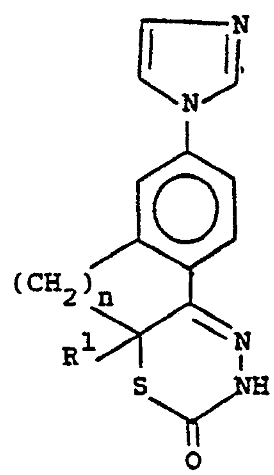

(1)

oder eines pharmazeutisch verträglichen Salzes davon, wobei:
$R^1$ ein Wasserstoffatom oder eine Methylgruppe bedeutet; und
n eins ist und wenn $R^1$ ein Wasserstoffatom ist, n auch zwei sein kann;
welches Verfahren umfaßt:
a) zur Herstellung einer Verbindung der Formel (1), in der $R^1$ ein Wasserstoffatom bedeutet, Umsetzung einer Verbindung der Formel (2)

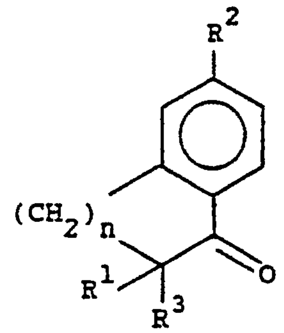

(2)

in der $R^1$ ein Wasserstoffatom bedeutet, $R^2$ eine 1-Imidazolylgruppe oder eine Vorstufe davon darstellt, $R^3$ ein Halogenatom bedeutet und n wie in Anspruch 1 definiert ist, mit einer Verbindung der Formel (3)

$R^4OCSNHNH_2$ (3)

in der $R^4$ einen $C_{1-4}$-Alkylrest darstellt; oder
b) Cyclisierung einer Verbindung der Formel (4)

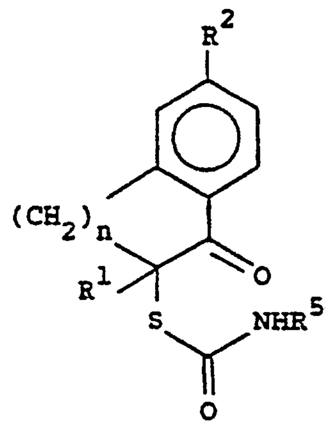

(4)

in der $R^1$ ein Wasserstoffatom oder eine Methylgruppe bedeutet und $R^2$ und n wie vorstehend definiert sind, und $R^5$ eine gegebenenfalls geschützte Aminogruppe darstellt, in Gegenwart einer Säure;
und danach falls erforderlich:
i) Umwandlung des Restes $R^2$ in die 1-Imidazolylgruppe,
ii) Entfernung irgendwelcher Schutzgruppen,
iii) Herstellung eines pharmazeutisch verträglichen Salzes.

2. Verfahren nach Anspruch 1 zur Herstellung einer Verbindung, in der n eins ist.

3. Verfahren nach Anspruch 1 oder 2 zur Herstellung einer Verbindung, in der $R^1$ ein Wasserstoffatom bedeutet.

4. Verfahren nach Anspruch 1 zur Herstellung einer Verbindung, nämlich:

7-(1H-Imidazol-1-yl)-9,9a-dihydroindeno[1,2-e]-[1,3,4]-thiadiazin-2(3H)-on,

7-(1H-Imidazol-1-yl)-9a-methyl-9,9a-dihydroindeno-[1,2-e]-[1,3,4]-thiadiazin-2(3H)-on oder

7-(1H-Imidazol-1-yl)-9,10-dihydro-10aH-naphtho[1,2-e]-[1,3,4]-thiadiazin-2(3H)-on,

oder ein pharmazeutisch verträgliches Salz davon.

5. Verfahren zur Herstellung eines Arzneimittels, umfassend das Zusammenbringen einer Verbindung der Formel (1) oder eines pharmazeutisch verträglichen Salzes davon gemäß Anspruch 1 und eines pharmazeutisch verträglichen Trägers.

6. Verfahren nach Anspruch 1, wobei die Verbindung der Formel (4) durch Umsetzung einer Verbindung der Formel (2)

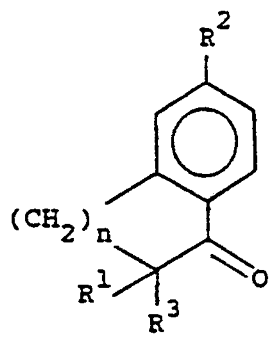

(2)

in der $R^1$ ein Wasserstoffatom oder eine Methylgruppe bedeutet, und $R^2$, $R^3$ und n wie in Anspruch 1 definiert sind, mit einer Verbindung der Formel (5)

$M^{\oplus}$ $^{\ominus}O\text{-}CS\text{-}NHR^5$ (5)

in der $R^5$ eine gegebenenfalls geschützte Aminogruppe bedeutet, und $M^+$ ein Gegenion darstellt, hergestellt wird.

7. Verfahren nach Anspruch 6, umfassend die Umsetzung von 2-Brom-5-(1H-imidazol-1-yl)-1-indanon mit Kaliumthiocarbazat und anschließend Behandlung mit Säure, wobei 7-(1H-Imidazol-1-yl)-9,9a-dihydroindeno-[1,2-e]-[1,3,4]-thiadiazin-2(3H)-on erhalten wird.

8. Verfahren zur Herstellung einer Verbindung der Formel (4)

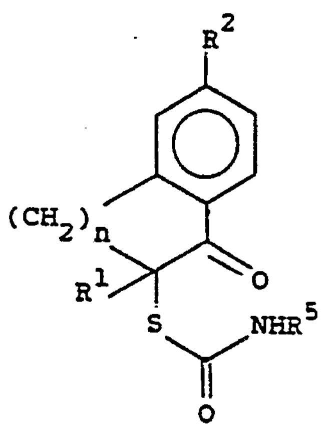

(4)

in der $R^1$ und n wie in Anspruch 1 definiert sind,
$R^2$ eine 1-Imidazolylgruppe bedeutet, und
$R^5$ eine gegebenenfalls geschützte Aminogruppe darstellt, umfassend die Umsetzung einer Verbindung der Formel (2)

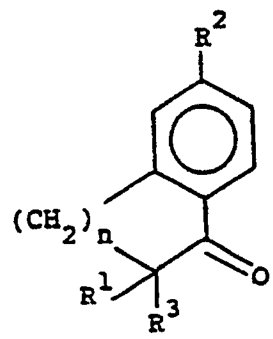

(2)

in der $R^1$, $R^2$ und n wie vorstehend definiert sind, und $R^3$ ein Halogenatom bedeutet, mit einer Verbindung der Formel (5)

$M^{\oplus}\,{}^{\ominus}O\text{-}CS\text{-}NHR^5$ (5)

in der $R^5$ eine gegebenenfalls geschützte Aminogruppe bedeutet, und $M^+$ ein Gegenion darstellt.

**9.** Verwendung einer Verbindung der Formel (1) gemäß Anspruch 1 oder eines pharmazeutisch verträglichen Salzes davon bei der Herstellung eines Arzneimittels zur Behandlung von Stauungsherzversagen.